# EUROPEAN PATENT APPLICATION

(11) **EP 1 244 037 A1**
(43) Date of publication of application: **25.09.2002**
(21) Application number: 01106384.9
(22) Date of filing: 19.03.2001
(51) Int. Cl.: G06F 17/60

(54) **Method, apparatus and product for automatically calculating a compensated annuitant mortality table**

(71) Applicant: GE Frankona Management Service GmbH, 81675 München (DE)
(72) Inventor: Sieger, Caspar, Dr. Dr., 82049 Pullach (DE); Pfeiffer, Thorsten, Dr., 55545 Bad Kreuznach (DE)
(74) Representative: VOSSIUS & PARTNER

(57) **Abstract**

This invention is related to a methods, an apparatus and a product for automatically calculating a compensated annuitant mortality table. The product and apparatus described utilises a method of automatically calculating a compensated annuitant mortality table, said method comprising the steps of: providing to a data processing means digitised demographic data representing past mortality rates q(T) for a plurality of years, T denoting the year; calculating the present mortality rates of the actual year based on said digitised demographic data; evaluating compensation rates based on medical advancements in at least one medical field and inputting said compensation rates into said data processing means; determining compensated mortality rates for the subsequent years by adjusting the present mortality rates based on said compensation rates. This invention can be used preferably for life annuity products

## Description

This invention is related to methods, an apparatus and a product for automatically calculating a compensated annuitant mortality table. The invention may be applied preferably to life annuity products.

In view of the present economic forecasts and the concern that in the future the government social security system may not be able to adequately fulfil the retirement needs of those entitled to benefits, there appears to be an increasing interest in private retirement programs. Statistical data indicate that the demand for private retirement programs, such as insurances offering retirement benefits, has increased dramatically in the past ten years. From 1989 to 1999, the German Insurance industry reported an increase of approximately 20 percent in the share of retirement insurance policies issued. Retirement insurance policies are generally referred to as annuity products. Normally, annuity products offer retirement funds and a particular guaranteed death benefit which varies depending on the annuity product purchased.

Annuity products enable the policy holder or the insured to accumulate funds for retirement. At retirement, the insured generally either receives a lump sum or receives regular payments for a period of time. In the case of life annuity products, the insured receives payments for an undetermined period of time, generally starting from a certain retirement age and ending when the insured dies. Thus, the projected costs incurred by the insurer of a life annuity are primarily dependent on the amount and duration of payments to be made, and the duration of the payments is determined by the life span of the individual insured. Since the life span of the insured is uncertain, there are several risks involved when estimating the projected costs of the life annuity. In order to accurately estimate these costs, it is necessary to be able to reliably predict the expected life span of the insured so that an adequate amount of funds can be reserved to cover these costs when they are due at some time in the future.

There are some conventional methods and apparatuses for calculating life expectancies or rather mortality rates of individuals. Presently, the most widely used technique is a typical projection method. Past mortality rates are interpolated and projected into the future. There are several problems which arise when using such methods. For example, one has to decide which interval from the past should be considered when estimating the future mortality rates and further what factor should be used in order to compensate for possible changes in the death rate. Since many relevant factors may also be ignored when strictly using such a method, the projected mortality rates obtained may represent an overestimate.

Another method used is a "targeting method". A sub group is selected as a "target group". The mortality rate of the target group is used to project the mortality rate for the entire population. However, the problem is that the target group has to be carefully selected; otherwise the data may be misleading.

It is quite important that the mortality data is accurately calculated. For example, a significant overestimate of the mortality rate could result in major financial repercussions. If the insured lives longer than expected, then the insurer or possibly reinsurance company may face a financial loss due to the unexpected annuity payments entitled to the insured.

Although there are several methods available for calculating mortality data, these methods rely on past data only and do not account for factors which may affect future mortality rates. Such factors include, e.g., the social-economic conditions of the insurer, differences between the population and the insured, etc.

Thus, there is a need for a method of automatically determining an improved mortality table that does not represent an overestimate, and a product or apparatus which utilises such an accurate method.

It is therefore an object of the present invention to provide an apparatus, product and more effective method for automatically calculating an improved annuitant mortality table.

The object of the present invention is achieved with the features of the claims.

The present invention is based on the idea to provide a compensated annuitant mortality table taking into account said factors and especially future advancements in the fields of medicine, hygiene, nutrition and accident prevention.

Reviewing the past mortality data, it can be observed that the average life span of the individual has increased remarkably over the last 100 years. In order to accurately predict future mortality rates, one has to consider the reasons for the increase in longevity. It is known that there is a direct correlation between medical advancements and the longevity of the individual. Through significant medical advancements life can be prolonged, e.g. by improved therapy thereby possibly eliminating or delaying an illness and by improved precautionary treatments or diagnostic tools thereby possibly avoiding a particular disease or illness due to early detection.

If the present mortality data is characterised into particular categories based on the major causes of death, the expected medical developments pertaining to the particular cause of death can be considered. Compensation rates can then be evaluated based on these expected medical developments and used for calculating future mortality rates or tables.

According to the invention, major causes of death considered herein are, e.g., cardiovascular diseases, all cancer, injuries and intoxication, etc.

Medical advancements that are taken into consideration according to the invention are listed in the following with reference to various aspects of said major causes of death:

### Cardiovascular diseases:

Hypertension:
New medications:
   New antihypertensive drugs (e.g., A II ― Antagonists, ANPase-, Endothelin-, Renin-Inhibitors, Potassium-Channel-Blockers, ERDF-Analoga) (longer acting, less side-effects, higher effectiveness)
Heart Insufficiency/congestive heart failure:
   New medications with better activity than ACE-Inhibitors and A II
   Antagonists
   New vasodilatators like ANPase-Inhibitors
   First new positive inotropic drugs
Heart infarction/coronary artery diseases:
   Better risk factor management (Better cholesterol lowering drugs/ HMG-CoA reductase inhibitors Cholesterol lowering - 50% - better blood pressure and diabetes control)
   Early detection with atherogenic marker and ultrasound
   Preventing restenosis by coated stents after PTCA
   Better antithrombotic drugs like thromboxane antagonists, Clopidogrel, Ticlopidine
   More effective thrombolytics
   Better PTCA techniques (coated stents) and minimal invasive CABG
Stroke:
   Better blood pressure control
   PTCA of the Aa. Carotis
   New A II-Antagonist passing easily the blood/brain barrier
   Centrally acting vasolilatators/cytoprotectors like nimodipine
Others (Valve defects, arrhythmia, endocarditis/angiitis):
   More effective antiarrhythmic drugs, better and smaller
   Cardioverters/Defibrillators,
   New antiinflammatory drugs

### All cancer:

New therapeutic tools:
Drug targeting by means of monoclonal antibodies allowing higher dosages with fewer side effects:
   New anticancer drugs like taxoteres, aromatase inhibitors and topoisomerase I inhibitors with higher efficacy against Mama and ovarian tumours
      New drugs to antagonise unintended cytotoxic effects, allowing higher dosages (e.g.: G-CSF, Myelopoietin, Cytokinines):
      More sophisticated therapeutic anticancer regimen including neoadiuvant radiation and/or combination chemotherapy, new therapy regimen and after surgery therapies.
New diagnostic tools:
   Detection of more gene loci, predicting an increased risk for cancer and allowing a much more effective screening
   Detection of new antibodies like CEA (Carcinoembryonal Antigen) or PSA (Prostate specific Antigen), allowing the early detection of primary or secondary tumour cells
   New Sonography devices with better resolution and 3-D imaging, new X-ray and NMR-devices
   Reduction of risk factors; for example, smoking will be slightly decreasing in men and remain constant in woman.

Another factor to be considered is the difference between the general population and the insured. Since the insured has decided to invest in such a retirement product, one can assume that the insured is in generally good health and expects to live long enough to reap the benefits of the investment. Therefore, the life span of the insured is essentially expected to be longer than the average of the general population. Therefore, a multiplication factor (fₓ) is used to compensate for this difference.

The subject-matter according to the present invention has the particular advantage that an annuitant mortality table can be calculated which considers primary factors which have been shown to have a direct impact on the mortality. Thus, financial risks involved due to an unexpected longevity of the insured resulting in a longer duration of annuity payments as originally predicted can be minimised.

Another advantage of the present invention exists in providing an innovative apparatus and product which is arranged to use a more accurate method of automatically calculating mortality rates.

One preferred embodiment of the present invention is a method of automatically calculating a compensated annuitant mortality table. The method comprising the steps of: a) providing to a data processing means digitised demographic data representing past mortality rates q(T) for a plurality of years, T denoting the year; b) calculating the present mortality rates of the actual year based on said digitised demographic data; c) evaluating compensation rates based on medical advancements in at least one medical field and inputting said compensation rates into said data processing means; and d) determining compensated mortality rates for the subsequent years by adjusting the present mortality rates based on said compensation rates.

Preferably, the digitised demographic data provided to said data processing means in step a) comprises mortality rates q_{x,i,s}(T) for age x, death cause i and sex s. The mortality rates q_{x,i,s}(T) are provided preferably for a plurality of years, in particular for the years from 1979 to the year of the latest available mortality rates.

The calculating step b) comprises preferably the steps of: i) identifying a plurality of main causes of death; ii) splitting said past mortality rates q(T) of a pre-selected year into percentages for each of said main causes of death identified in step i); iii) projecting said past mortality rates q(T) for a plurality of years to the present year for obtaining the mortality rates q(T) for the present year; and iv) splitting the mortality rates obtained in step iii) into by-cause rates based on said percentages calculated in step ii).

In step b)iii), said mortality rates are projected preferably by fitting a linear curve to In(qₓ) in order to determine a trend, and then extrapolating to the present year.

The evaluation of compensation rates considers preferably at least one of new therapeutic tools, new diagnostic tools and/or a reduction of risk factors. Preferably, the evaluating step comprises the step of searching, screening and evaluating patent applications and/or publications in at least one medical field.

Preferably, step d) comprises the steps of: i) calculating said compensated mortality rates q_{x,i,s}(T) for future pivot years by the formula q_{x,i,s}(T) = q_{x,i,s}(present year) ∗ (1 - rate_{i,T,s}), rate_{i,T,s} representing the evaluated medical advancements; and ii) calculating the compensated mortality rate for each year by the formula q_{x,s} (T) = Σᵢ q_{x,i,s}(T). Intervals of ten years are used preferably between said pivot years.

Preferably, the method of the present invention further comprises a step of graduating the compensated mortality rates for the future years.

Preferably, the method of the present invention further comprises a step of extrapolating the age range to age 140.

The method of the present invention may further comprise a step of adjusting for the difference between the mortality of the population and the mortality of insured lives by applying a multiplication factor fₓ.

Preferably, the method of the present invention further comprises a step of applying a security allowance for the risk of fluctuations by subtracting a safety factor sₓ from the mortality rates.

In order to assure that funds are available for annuity payments to the insured at a particular year in the future, insurers are obligated by law to have a reserve to account for the annuity payments. The amount of the reserve necessary is determined by using standardised actuarial tables. Since standardised actuarial tables generally do not account for various factors such as future medical advancements, there is a chance that the mortality rate of the insured is an overestimate. As a result, an insurer may need more funds to pay the annuity payments as is available in the reserve. Therefore, insurers commonly rely on re-insurance companies to provide security against such a possible financial loss. In order to predict the actual amount necessary for covering the possible financial loss to the insurer, re-insurance companies need a method of calculating a compensated reserve based on method which is more accurate than and a considerable improvement over the standardized actuarial tables.

Thus, another preferred embodiment of the present invention is a method of automatically calculating a compensated reserve using the compensated annuitant mortality table obtained by the method of the present invention, for example by the method of the aforementioned preferred embodiment.

This method comprises preferably the steps of: calculating a first reserve for a particular year T using a standard annuitant mortality table based on age and sex; calculating a second reserve for said particular year T using the compensated annuitant mortality table based on age, sex and death cause; and calculating a difference between said first and said second reserves, said difference being said compensated reserve.

Another embodiment of the present invention is an apparatus for automatically calculating a compensated annuitant mortality table comprising: a data processing means which is arranged to receive information into a memory, said information comprising digitised demographic data representing past mortality rates q(T) for a plurality of years, T denoting the year. The data processing means includes a processor which comprises: mortality rates calculating means for calculating the present mortality rates of the actual year based on said digitised demographic data; evaluation input means for inputting evaluated compensation rates based on medical advancements in at least one medical field; and compensated mortality rates determination means for determining compensated mortality rates for the following years by adjusting the present mortality rates based on said compensation rates, wherein said apparatus is arranged to produce, in use, two-dimensional tables of graduated mortality rates for males and females for a plurality of future years.

Preferably, the data processing means is arranged to receive mortality rates q_{x,i,s}(T) for age x, death cause i and sex s. The mortality rates q_{x,i,s}(T) are provided preferably for a plurality of years, in particular for the years from 1979 to the year of the latest available mortality rates q_{x,i,s}(T).

Preferably, the mortality rates calculating means is arranged to identify a plurality of main causes for death; split said past mortality rates q(T) of a pre-selected year into percentages for each of said identified main causes for death; project said past mortality rates q(T) for a plurality of years to the present year; and split the projected present mortality rates into by-cause rates based on said calculated percentages.

The evaluation input means is arranged preferably to receive compensation data representing medical advancements that consider at least one of new therapeutic tools, new diagnostic tools and/or a reduction of risk factors.

The apparatus of the present invention may further comprise means for searching, screening and evaluating patent applications and/or publications in at least one medical field.

Preferably, the compensated mortality rate determination means is arranged to calculate said compensated mortality rates q_{x,i,s}(T) for future pivot years by the formula q_{x,i,s}(T) = q_{x,i,s}(present year) ∗ (1 - rate_{i,T,s}), rate_{i,T,s} representing the evaluated medical advancements; and calculate the compensated mortality rate q_{x,s}(T) for each year by the formula q_{x,s}(T) = Σᵢ q_{x,i,s} (T).

The apparatus of the present invention may further comprise a means for automatically calculating a compensated reserve using the compensated annuitant mortality table. The means for automatically calculating a compensated reserve is arranged to calculate a first reserve for a particular year T using a standard annuitant mortality table based on age and sex; to calculate a second reserve for said particular year T using the compensated annuitant mortality table obtained based on age, sex and death cause; and then to calculate a difference between said first and said second reserves, said difference being said compensated reserve.

Another embodiment of the invention is a computer program product comprising program code means stored on a computer readable storage medium for performing the method of any of aforementioned embodiments when the product is run on a computer.

The various embodiments presented in the specification are used for the sake of description and clarification of the invention, and thus should not be interpreted as limiting the scope of the invention as such. The invention is represented by the claims and any obvious modifications thereof.

## Claims

1. Method of automatically calculating a compensated annuitant mortality table, said method comprising the steps of:
a) providing to a data processing means digitised demographic data representing past mortality rates q(T) for a plurality of years, T denoting the year;
b) calculating the present mortality rates of the actual year based on said digitised demographic data;
c) evaluating compensation rates based on medical advancements in at least one medical field and inputting said compensation rates into said data processing means;
d) determining compensated mortality rates for the subsequent years by adjusting the present mortality rates based on said compensation rates.

2. The method of claim 1, wherein said digitised demographic data provided to said data processing means in step a) comprises mortality rates q_{x,i,s}(T) for age x, death cause i and sex s.

3. The method of claim 2, wherein said mortality rates q_{x,i,s}(T) are provided for a plurality of years, in particular for the years from 1979 to the year of the latest available mortality rates.

4. The method of claim 1, 2 or 3, wherein said calculating step b) comprises the steps:
i) identifying a plurality of main causes for death;
ii) splitting said past mortality rates q(T) of a preselected year into percentages for each of said main causes for death identified in step i);
iii) projecting said past mortality rates q(T) for a plurality of years to the present year for obtaining the mortality rates q(T) for the present year; and
iv) splitting the mortality rates obtained in step iii) into by-cause rates based on said percentages calculated in step ii).

5. The method of any of claims 1 to 4, wherein said evaluation of compensation rates considers at least one of new therapeutic tools, new diagnostic tools, reduction of risk factors.

6. The method of any of claims 1 to 5, wherein said evaluating step comprises the step of searching, screening and evaluating patent applications and/or publications in said at least one medical field.

7. The method of any of claims 1 to 6, wherein step d) comprises the steps of:
i) calculating said compensated mortality rates q_{x,i,s}(T) for future pivot years by the formula q_{x,i,s}(T) = q_{x,i,s}(present year) ∗ (1 - rate_{i,T,s}), rate_{i,T,s} representing the evaluated medical advancements; and
ii) calculating the compensated mortality rate q_{x,s} (T) for each year by the formula q_{x,s} (T) = Σᵢ q_{x,i,s}(T).

8. The method of claim 7, wherein steps of ten years are between said pivot years.

9. The method of any of claims 4 to 8, wherein in step b)iii) said mortality rates are projected by fitting a linear curve to In(qₓ) to determine a trend, and then extrapolating to the present year.

10. The method of any of claims 1 to 9, further comprising the step of graduating the compensated mortality rates for the future years.

11. The method of any of claims 1 to 10, further comprising the step of extrapolating the age range to age 140.

12. The method of any of claims 1 to 11, further comprising the step of adjusting for the difference between population and insured lives mortality by applying a multiplication factor fₓ.

13. The method of any of claims 1 to 12, further comprising the step of applying a security allowance for the risk of fluctuations by subtracting a safety factor sₓ from said mortality rates.

14. A method of automatically calculating a compensated reserve using the compensated annuitant mortality table obtained by a method according to any of claims 1 to 13.

15. The method of claim 14, comprising the steps of:
- calculating a first reserve for a particular year T using a standard annuitant mortality table based on age and sex;
- calculating a second reserve for said particular year T using the compensated annuitant mortality table obtained using the method of any of claims 1 to 13 based on age, sex and death cause; and
- calculating a difference between said first and said second reserves, said difference being said compensated reserve.

16. Apparatus for automatically calculating a compensated annuitant mortality table comprising:
a data processing means which is arranged to receive information into a memory, said information comprising digitised demographic data representing past mortality rates q(T) for a plurality of years, T denoting the year, said data processing means including a processor which comprises:
A) mortality rates calculating means for calculating the present mortality rates of the actual year based on said digitised demographic data;
B) evaluation input means for inputting evaluated compensation rates based on medical advancements in at least one medical field;
C) compensated mortality rates determination means for determining compensated mortality rates for the following years by adjusting the present mortality rates based on said compensation rates;
said apparatus being arranged to produce, in use, two-dimensional tables of graduated mortality rates for males and females for a plurality of future years.

17. The apparatus as claimed in claim 16, wherein said data processing means is arranged to receive mortality rates q_{x,i,s}(T) for age x, death cause i and sex s.

18. The apparatus as claimed in claim 17, wherein said mortality rates q_{x,i,s}(T) are provided for a plurality of years, in particular for the years from 1979 to the year of the latest available mortality rates q_{x,i,s}(T).

19. The apparatus as claimed in claim 16, 17 or 18, wherein said mortality rates calculating means is arranged to
identify a plurality of main causes for death;
split said past mortality rates q(T) of a preselected year into percentages for each of said identified main causes for death;
project said past mortality rates q(T) for a plurality of years to the present year; and
split the projected present mortality rates into by-cause rates based on said calculated percentages.

20. The apparatus of any of claims 16 to 19, wherein said evaluation input means is arranged to receive compensation data representing medical advancements that consider at least one of new therapeutic tools, new diagnostic tools, reduction of risk factors.

21. The apparatus of any of claims 16 to 20, further comprising means for searching, screening and evaluating of patent applications and/or publications in said at least one medical field.

22. The apparatus of any of claims 16 to 21, wherein said compensated mortality rates determination means is arranged to
calculate said compensated mortality rates q_{x,i,s}(T) for future pivot years by the formula q_{x,i,s}(T) = q_{x,i,s}(present year) ∗ (1 - rate_{i,T,s}), rate_{i,T,s} representing the evaluated medical advancements; and
calculate the compensated mortality rate q_{x,s}(T) for each year by the formula q_{x,s}(T) = Σᵢ q_{x,i,s} (T).

23. The apparatus of any of claims 16 to 22, further comprising a means for automatically calculating a compensated reserve using the compensated annuitant mortality table obtained by a method according to any of claims 1 to 13.

24. The apparatus of claim 23, wherein said means for automatically calculating a compensated reserve is arranged to
calculate a first reserve for a particular year T using a standard annuitant mortality table based on age and sex;
calculate a second reserve for said particular year T using the compensated annuitant mortality table obtained using the method of any of claims 1 to 13 based on age, sex and death cause; and
calculate a difference between said first and said second reserves, said difference being said compensated reserve.

25. A computer program product comprising program code means stored on a computer readable storage medium for performing the method of any of the claims 1 to 15 when said product is run on a computer.

26. Use of a method according to any of claims 1 to 15 or of an apparatus according to any of claims 16 to 24 or a computer program product according to claim 25 for calculating provision for each insured life of an insurance company.
